# FASCICULE DE BREVET EUROPEEN

(11) **EP 1 539 738 B1**
(45) Date de publication et mention de la délivrance du brevet: **04.03.2009**
(21) Numéro de dépôt: 03760060.8
(22) Date de dépôt: 18.06.2003
(51) Int. Cl.: C07D 401/12, A61K 31/506, A61P 25/00

(54) **NOUVEAUX DERIVES D'ARYL-4-HALOGENO-4- HETEROARYLMETHYLAMINO)-METHYL -PIPERIDIN-1-YL-METHANONE,LEUR PROCEDE DE PREPARATION ET LEUR UTILISATION A TITRE DE MEDICAMENTS**
NEUE ARYL-4-HALOGEN-4-(HETEROARYLMETHYLAMINO)METHYL PIPERIDIN-1-YL-METHANONDERIVATE, HERSTELLUNGSVERFAHREN UND DEREN VERWENDUNG ALS MEDIKAMENTE
NOVEL ARYL- 4-HALO-4- HETEROARYLMETHYLAMINO)-METHYL -PIPERIDIN-1-YL -METHANONE DERIVATIVES, METHODS FOR PRODUCTION AND USE THEREOF AS MEDICAMENTS

(30) Priorité: 18.06.2002 FR 0207470
(43) Date de publication de la demande: 15.06.2005
(73) Titulaire: PIERRE FABRE MEDICAMENT, 92100 Boulogne-Billancourt (FR)
(72) Inventeur: VACHER, Bernard, F-81100 Castres (FR); BONNAUD, Bernard, F-81090 Lagarrigue (FR); MAUREL, Jean-Louis, F-81100 Castres (FR); COLPAERT, Francis, F-81700 Puylaurens (FR)
(74) Mandataire: Ahner, Francis
(86) Numéro de dépôt international: PCT/FR2003/001873
(87) Numéro de publication internationale: WO 2003/106449

(56) Documents cités:
- WO-A-98/22459

## Description

Les agonistes 5-HT1_{A} peuvent être utile pour le traitement de certains désordres du système nerveux central (CNS Drugs 1998, 10(5), 343-353). Bien qu'un très grand nombre de composés aient été revendiqués comme ayant des propriétés agonistes au niveau des récepteurs du sous-type 5-HT1_{A}, deux seulement sont disponibles cliniquement (i.e., la buspirone : Europe et Etats-Unis et la tandospirone : Japon). Ces deux composés appartiennent, toutefois, à la même famille chimique (i.e., arylpipérazine) et présentent des profils pharmacologiques relativement similaires.

Le contraste entre le nombre de candidats et le nombre de composés disponibles cliniquement illustre, entre autres, les limites de la caractérisation pharmacologique des agonistes 5-HT1_{A} au moyen des critères tels que l'affinité, la sélectivité et des critères pharmacodynamiques classiques. L'efficacité des ligands au niveau des récepteurs 5-HT1_{A} n'est, par contre, que rarement rapportée. Or, il apparaît de plus en plus clairement que l'activité intrinsèque d'un ligand donc son efficacité au niveau des récepteurs 5-HT1_{A}, détermine non seulement son domaine d'activité thérapeutique dans le champ des indications 5-HT1_{A} potentielles mais encore son niveau d'activité dans une indication thérapeutique donnée (Eur. J. Pharmacol. 2001, 420, 103-112). Si, en théorie, l'activité intrinsèque (et donc l'efficacité) d'un ligand 5-HT1_{A} constitue un paramètre essentiel, en pratique, sa mesure reste dépendante des conditions expérimentales utilisées. Cette situation a, néanmoins, été mise à profit et est à la base du développement de systèmes dans lesquels l'efficacité relative des ligands 5-HT1_{A} peut être évaluée avec un pouvoir de résolution accru dans certaines régions du spectre d'activité intrinsèque (J. Pharmacol. Exp. Ther. 2000, 292(2), 684-91 ; Naunyn-Schmiedeberg's Arch. Pharmacol. 1997, 356, 551-61). L'utilisation desdits systèmes met en évidence deux éléments importants : l'étendue du domaine d'efficacité inexploré entre antagoniste et agoniste complet est vaste ; très peu de ligands possèdent une efficacité supérieure à celle de la 8-OH-DPAT (8-hydroxy-2-di-n-propylamiinotétraline) et ce malgré la profusion de ligands revendiqués comme 5-HT1_{A} agonistes. Il est remarquable, par exemple, que la 8-OH-DPAT, considérée comme l'agoniste 5-HT1_{A} de référence, soit dotée d'une efficacité médiocre comparée à celle de la sérotonine.

Compte tenu du potentiel thérapeutique important des composés dotés d'une activité agoniste pour les récepteurs 5-HT1_{A} et de l'absence de ligands dont l'efficacité se rapproche de celle de la sérotonine, la découverte de structures nouvelles possédant des propriétés agoniste 5-HT1_{A} supérieures à celle des ligands connus est fortement souhaitable. La demanderesse a découvert que plusieurs composés dérivés d'aryl-{4-halogéno-4-[(hétéroaryl-méthylamino)-méthyl]-pipéridin-1-yl}-méthanone interagissent sélectivement avec les récepteurs sérotoninergiques du sous-type 5-HT1_{A} au niveau desquels ils se comportent comme des agonistes efficaces. En tant que tel, les composés de l'invention sont donc potentiellement utiles pour le traitement des désordres sensibles à une régulation sérotoninergique contrôlée par les récepteurs 5-HT1_{A}. La liste des troubles, désordres et pathologies considérées comme sensibles à une telle régulations est longue, nous limitons toutefois le champ d'application de la présente invention au traitement de la dépression, de la dépendance à certaines substances et de la douleur.

L'état de la technique le plus proche est représenté par des composés du type pyridyn-2-yl-méthylamine (WO 98/22459) répondant à la formule suivante : 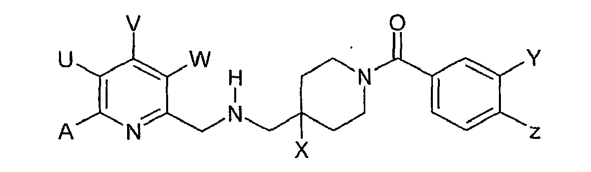 dans laquelle :
A représente, entre autres, un atome d'hydrogène ;
U représente, entre autres, un radical méthyle ;
V représente, entre autres, un atome d'hydrogène ;
W représente, entre autres, un atome d'hydrogène ;
X représente, entre autres, un atome de fluor ;
Y représente, entre autres, un atome de chlore ;
Z représente, entre autres, un atome d'hydrogène, de fluor ou de chlore.

Les composés en question sont revendiqués comme étant des agonistes 5-HT_{1A} sélectifs, utiles comme antidépresseurs ou analgésiques.
Les composés revendiquée dans le brevet WO 98/22459 et les composés de la présente invention se différencient donc par la nature de leur hétérocycle azoté et/ou la nature des substituants portés par ce dernier et/ou par la nature de l'atome d'halogène en position 4 du cycle pipéridinique et/ou la nature des substituants portés par le groupe aryl. Les composés de l'invention comme ceux revendiqués dans le brevet WO 98/22459 possèdent une forte affinité et sont sélectifs pour les récepteurs 5-HA1_{A} (vis à vis, en particulier, des récepteurs dopaminergique du sous-type D₂). Cependant, de manière tout à fait surprenante, les modification structurales introduites au niveau des composés de l'invention leur confèrent une efficacité généralement supérieure à celles des composés décrits dans WO 98/22459. Ainsi, à affinité et sélectivité quasi-équivalente, nous montrons, in vitro, que la capacité de plusieurs composés de l'invention à activer un complexe protéique effecteur est supérieure à celle du (3-chloro-4-fluoro-phényl)-(4-fluoro-4-{[(5-méthyl-pyridin-2-ylméthyl)-amino]-méthyl}-pipéridin-1-yl)-méthanone (composé I-66), l'agoniste le plus efficace décrit dans le brevet WO 98/22459. L'intérêt majeur des composés de l'invention réside donc dans leur capacité particulière et, à ce jour inégalée, à activer les récepteurs du sous-type 5-HT1_{A}; cette propriété est avantageuse car elle ouvre des perspectives thérapeutiques nouvelles en clinique humaine dans des domaines pour lesquels il existe un besoin thérapeutique important et pour lesquels les agonistes 5-HT1_{A} disponibles cliniquement ne sont pas efficaces, tels que, par exemple, le traitement de la dépression, de la dépendance à certaines substances ou de la douleur.

Plus spécifiquement, la présente invention se rapporte à de nouveaux dérivés aryl-{4-halogéno-4-[(hétéroaryl-méthylamino)-méthyl]-pipéridin-1-yl}-méthanone qui, sous forme de base, répondent à la formule générale (1) : 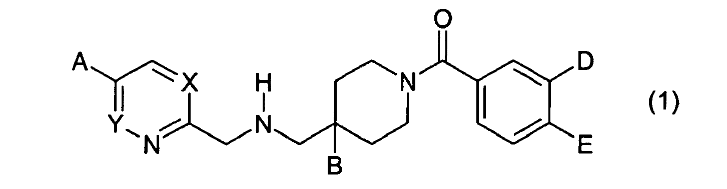 dans laquelle :
X représente un atome de carbone lié à un atome d'hydrogène (CH) ou un atome d'azote ;
Y représente un atome de carbone lié à un atome d'hydrogène (CH) ou un atome d'azote ;
A représente un radical methyle (CH₃), fluoromethyle (CH₂F), cyano (CN), hydroxyle (OH), méthoxyle (OCH₃), un atome de chlore ou un atome de fluor à la condition toutefois, que lorsque A est un radical méthyle (CH₃), et X et Y représentent simultanément un atome de carbone lié à un atome d'hydrogène, alors, B représente nécessairement un atome de chlore ;
B représente un atome de chlore ou un atome de fluor ;
D représente un atome d'hydrogène, un atome de chlore, un atome de fluor, un groupe cyano (C≡N) ou un groupe trifluorométhyle (CF₃) ;
E représente un atome d'hydrogène, de fluor ou de chlore ;
leurs sels d'addition et éventuellement les hydrates des sels d'addition avec les acides minéraux ou les acides organiques pharmaceutiquement acceptables ainsi que leurs formes tautomères.

La présente invention a en particulier pour objet les composés tels que définis dans la revendication 1.

L'invention vise en particulier les composés de formule générale (1) dans laquelle :
B et E représentent chacun un atome de fluor ;
D représente un atome de chlore.

Les dérivés de formule générale (1) peuvent être obtenus par le procédé décrit dans le schéma A.

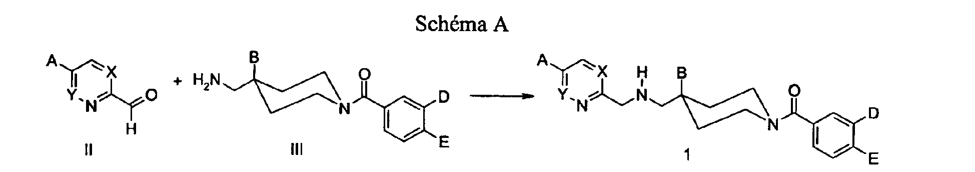

### Schéma A

Les composés de formule (1) peuvent être préparés par une réaction d'amination réductrice entre l'aldéhyde de formule (II) et l'amine primaire de formule (III) selon une méthode analogue à celle décrite dans le brevet WO 98/22459. Les composés de formule (1) sont purifiés suivant une ou plusieurs méthodes choisies parmi la cristallisation et/ou les techniques chromatographiques en phase liquide. Ils peuvent être, ensuite, si on le désire, salifiés au moyen d'un acide minéral ou organique pharmaceutiquement acceptable.

La préparation des aldéhydes de formule (II) dépend de la nature des groupes X,Y et A. La préparation des aldéhydes (IIa1-6) dans lesquels X et Y sont, ensemble, un groupe CH et A représente un groupe CH₃, CN, OCH₃, OPMB (l'abréviation 'PMB' signifie p-methoxybenzyl), un atome de chlore ou un atome de fluor, est décrite dans la littérature.

Ainsi, l'aldéhyde (IIa-1) dans lequel A est un groupe CH₃ peut être préparé selon la méthode décrite dans Arch. Pharm. (Weinheim, Ger.) 1977, 310(2), 128-36.

L'aldéhyde (IIa-2) dans lequel A est un groupe CN peut être préparé selon WO 98/16526.

Les aldéhydes (IIa-3) et (IIa-4) dans lequel A est un groupe OCH₃ ou OPMB respectivement, peuvent être préparés selon Tetrahedron: Asymmetry 2001, 12, 1047-51.

Les aldéhydes (IIa-5) et (IIa-6) dans lequel A est un atome de chlore ou un atome de fluor respectivement, peuvent être préparés selon J. Med. Chem. 1970, 13(6), 1124-30.

L'aldéhyde (IIa-7) dans lequel A est un groupe CH₂F est, quand à lui, préparé selon le procédé décrit dans le schéma B.

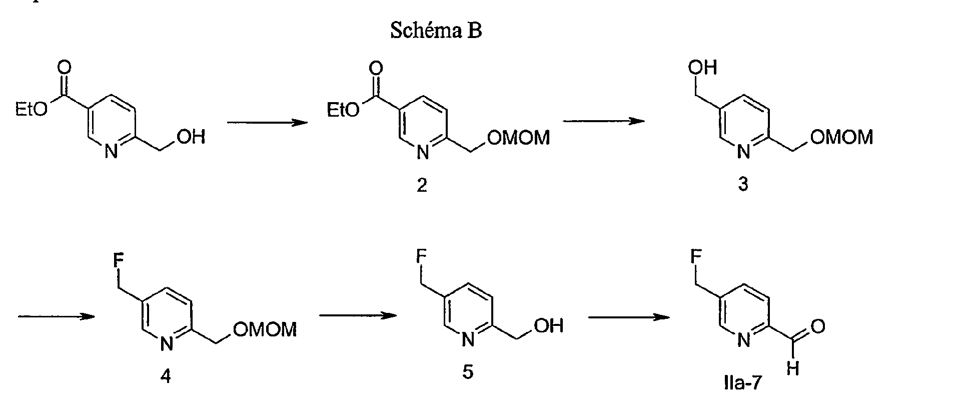

### Schéma B

La préparation de l'aldéhyde (IIa-7) utilise comme matière première l'ester éthylique de l'acide 6-hydroxyméthylnicotinique *(*Bioorg. Med. Chem. Lett. 1996, 6(24), 3025-28). La fonction alcool primaire est protégé sous forme d'éther de méthoxyméthyle puis la fonction ester du composé de formule (2) réduite au moyen d'hydrure de lithium aluminium pour donner l'alcool de formule (3). L'atome de fluor est introduit à partir de la fonction hydroxyle au moyen de morpholino-trifluorosulfure en présence du complexe HF-pyridine dans le dichlorométhane à basse température. Le clivage de la fonction ether méthoxyméthylique du composé de formule (4), effectué en milieu acide, conduit à l'alcool de formule (5) qui est ensuite oxydé en l'aldéhyde désiré (IIa-7) au moyen de dioxyde de manganèse (MnO₂) selon une méthode analogue à celle décrite dans WO 98/22459.

La préparation de l'aldéhyde (IIb) dans lesquels X est un atome d'azote, Y est un groupe CH et A représente un groupe CH₃ est décrite dans le brevet US 4,923,989.

L'aldéhyde (IIc) dans lesquels X est un groupe CH, Y est un atome d'azote et A représente un groupe CH₃ peut être préparé à partir de la 6-methyl-pyridazine-3-carbonitrile (Heterocycles 1986, 24(3), 793-7) par réduction de la fonction cyano au moyen d'hydrure de diisobutylaluminium dans le tétrahydrofurane à basse température selon une méthode classique bien connue du chimiste organicien.

La préparation des amines primaires de formule (IIIa) dans laquelle B est un atome de fluor, D et E ont la même signification que précédemment, est effectuée selon une méthode analogue à celle décrite dans WO 98/22459 et J. Med. Chem. 1999, 42(9), 1648-60.

Les amines primaires de formule (IIIb) dans laquelle B est un atome de chlore, D et E ont la même signification que précédemment, sont préparées selon une méthode similaire à celle des amines primaires de formule (IIIa), J. Med. Chem. 1999, 42(9), 1648-60 et WO 98/22459. Cependant, l'étape d'ouverture du spiro-époxyde est effectuée au moyen d'une solution d'acide chlorhydrique (4M) dans le dioxane et non par le complexe acide fluorhydrique-pyridine comme dans la synthèse des amines du type (IIIa). Les acides benzoïques utilisés comme produits de départ dans la préparation des dit spiro-époxydes sont disponibles commercialement ; excepté l'acide 3-cyano-4-fluoro-benzoïque qui peut être préparé selon la méthode décrite dans Tetrahedron Lett. 1997, 38(18), 3131-34.

L'invention a aussi pour objet les compositions pharmaceutiques contenant à titre de principe actif au moins un des dérivés de formule générale (1) ou un de ses sels ou hydrates de ses sels en combinaison avec un ou plusieurs supports inertes ou autres véhicules pharmaceutiquement acceptables.

Les compositions pharmaceutiques selon l'invention peuvent être, à titre d'exemple, des compositions administrables par voie orale, nasale, sublinguale, rectale ou parentérale. A titre d'exemple de compositions administrables par voie orale on peut citer les comprimés, les gélules, les granules, les poudres et les solutions ou suspensions orales.

Les formulations appropriées pour la forme d'administration choisie sont connues et décrites, par exemple dans : Remington, The Science and Practice of Pharmacy, 19ième édition, 1995, Mack Publishing Company.

La dose efficace d'un composé de l'invention varie en fonction de nombreux paramètres tels que, par exemple, la voie d'administration choisie, le poids, l'âge, le sexe, l'état d'avancement de la pathologie à traiter et la sensibilité de l'individu à traiter. En conséquence, la posologie optimale devra être déterminée par le spécialiste en la matière en fonction des paramètres qu'il juge pertinents. Bien que les doses efficaces d'un composé de l'invention puissent varier dans de larges proportions, les doses journalières pourraient s'échelonner entre 0,01 mg et 100 mg par Kg de poids corporel de l'individu à traiter. Une dose journalière d'un composé de l'invention comprise entre 0,05 mg et 50 mg par Kg de poids corporel de l'individu à traiter étant, toutefois, préférée.

Les compositions pharmaceutiques selon l'invention sont utiles dans le traitement de la dépression, de la dépendance à certaines substance et de la douleur.

### Exemples

Les exemples suivants illustrent l'invention mais ne la limitent en aucune façon.

Dans les exemples ci-après :
(i) l'avancement des réactions est suivi par chromatographie sur couche mince (CCM) et par conséquent les temps de réaction ne sont mentionnés qu'à titre indicatif ;
(ii) des formes cristallines différentes peuvent donner des points de fusion différents, les points de fusion rapportés dans la présente demande sont ceux des produits préparés selon la méthode décrite et ne sont pas corrigés ;
(iii) la structure des produits obtenus selon l'invention est confirmée par les spectres de résonance magnétique nucléaire (RMN) et l'analyse centésimale, la pureté des produits finaux est vérifiée par CCM ;
(iv) les spectres RMN sont enregistrés dans le solvant indiqué. Les déplacements chimiques (δ) sont exprimés en partie par million (ppm) par rapport au tétraméthylsilane. La multiplicité des signaux est indiquée par : s, singulet ; d, doublet ; t, triplet ; q, quadruplet ; m, multiplet ; 1, large ;
(v) les différents symboles des unités ont leur signification habituelle : µg (microgramme) ; mg (milligramme) ; g (gramme) ; ml (millilitre) ; °C (degré Celsius) ; mmole (millimole) ; nmole (nanomole) ; cm (centimètre) ; nm (nanomètre) ; min (minute) ; les pressions sont données en millibars (mb) ;
(vi) les abréviations ont la signification suivante : F (point de fusion) ; Eb (point d'ébullition) ; par "température ambiante" on entend une température comprise entre 20°C et 25°C.

### Préparation de l'intermédiaire (IIa-7)

### Etape 1 : Ester éthylique de l'acide 6-méthoxyméthoxyméthyl-nicotinique (2)

Dans une suspension d'hydrure de sodium (8 g, 1,98 10⁻¹ moles) dans du DMF (250 ml) refroidie à -15°C et maintenue sous atmosphère d'azote, on ajoute goutte à goutte l'ester éthylique de l'acide 6-hydroxyméthyl-nicotinique (30 g, 1,65 10⁻¹ moles) et le mélange est agité 10 min puis refroidi à -23°C. On additionne alors goutte à goutte le chlorométhylméthyléther, le mélange est agité pendant 5 minutes après la fin de l'addition puis versé dans 600 ml d'une solution saturée de NaHCO₃ glacée. Le mélange est extrait à l'éther de pétrole, les phases organiques combinées sont lavées à l'eau jusqu'à neutralité, puis à l'eau salée et séchées sur Na₂SO₄. Après filtration, le solvant est évaporé sous vide et le résidu distillé (P : 4.2 10⁻² mb; Eb : 120-130 °C). On obtient le produit du titre sous la forme d'une huile jaune (21 g) qui est utilisée dans l'étape suivante sans autre purification.

### Etape 2 : (6-Méthoxyméthoxyméthyl-pyridin-3-yl)-méthanol (3)

Dans une suspension d'hydrure de lithium aluminium (9 g, 2.18 10⁻¹ moles) dans du tétrahydrofurane (200 ml) refroidie à -80°C et maintenue sous atmosphère inerte est ajouté goutte à goutte le composé (2) dissous dans du tetrahydrofurane (100 ml). Le mélange réactionnel est agité à -80°C pendant 30 min après la fin de l'addition. On ajoute H₂O (18 ml), NaOH à 10% dans l'eau (23 ml), THF (200 ml) puis H₂O (53 ml). La température est alors ramenée à la température ambiante puis on ajoute à la suspension de l'acétate d'ammonium (20 g, 2.6 10⁻¹ moles). Après 15 minutes, le mélange réactionnel est filtré sur célite et le filtrat concentré sous-vide. Le résidu est repris dans du dichlorométhane, décanté et la phase organique séchée sur Na₂SO₄. Après concentration sous pression réduite, le résidu est purifié par filtration sur silice (éluent : CH₂Cl₂ / méthanol, 96 : 4). Le produit obtenu (14.2 g) est utilisé dans l'étape suivante sans autre purification.

### Etape 3 : 5-Fluorométhyl-2-méthoxyméthoxyméthyl-pyridine (4)

Dans une solution de morpholino-sulfurlrifluoride (2 ml, 1.63 10⁻² moles) dans du dichlorométhane (32 ml) refroidie à -78°C et maintenue sous atmosphère inerte, on additionne une solution du complexe HF-pyridine à 70% (0.3 ml) puis goutte à goutte 1 g (5.4 mmoles) du composé (3) en solution dans du CH₂Cl₂ (10 ml). Le mélange est agité 2 heures à-78°C puis versé dans une solution saturée de NaHCO₃ (75 ml). La phase aqueuse est extraite au dichlorométhane, les phases organiques combinées sont lavées à l'eau salée, séchées sur Na₂SO₄, filtrées et concentrées sous vide. Le résidu est distillé boule à boule (P : 4.7 10⁻² mb, Eb : 110-130 °C). Le composé du titre est isolé sous forme d'une huile incolore (0.3 g) utilisée directement dans l'étape suivante.

### Etape 4 : (5-Fluorométhyl-pyridine-2-yl)-méthanol (5).

Dans une solution d'acide chlorhydrique (4.2 N) dans l'éthanol (5.5 ml) refroidie à 0°C on additionne goutte à goutte 0.45 g de composé (4) en solution dans l'éthanol. A la fin de l'addition, la température du mélange est ramenée à la température ambiante et l'agitation poursuivie pendant 12 heures. Le mélange réactionnel est concentré sous-vide et le résidu obtenu lavé à l'éther d'isopropyle. La phase éther est éliminée et le résidu repris dans une solution CH₂Cl₂ / méthanol (75 : 25) puis agité sur Na₂CO₃ (1.2 .g). Le mélange est filtré et le filtrat évaporé pour donner une huile incolore (0.3 g) qui est utilisée sans autre purification dans l'étape suivante.

### Etape 5 : 5-Fluorométhyl-pyridine-2-carbaldéhyde (IIa-7)

Dans une solution de (0.3 g) de composé (5) dans le chloroforme (5 ml) à température ambiante on ajoute en une fraction (1.4 g) de dioxyde de manganèse. La suspension est agitée vigoureusement pendant 7 heures puis diluée avec du chloroforme et filtrée sur célite. Le filtrat est concentré sous vide et l'huile jaune obtenue (0.25g) est utilisée en l'état dans l'étape suivante d'amination réductrice.
¹HRMN (CDCl₃) δ : 5:53 (d, 2H); 7.90 (d, 1H); 8.01 (d, 1H); 8.79 (s, 1 H); 10.09 (s, 1 H).

### Exemple comparatif 1 : préparation du (3-Chloro-4-fluoro-phényl)-(4-fluoro-4-{[(5-hydroxy-pyridin-2-ylméthyl)-amino]-méthyl}-pipéridin-1-yl)-méthanone (1-1)

### Etape 1 : préparation du (4-{[(5-Benzyloxy-pyridin-2-ylméthyl)-amino]-méthyl}-4-fluoro-pipéridin-1-yl)- (3-chloro-4-fluoro-phényl)-méthanone (1-1a)

Dans une solution de 2 g (8.22 mmoles) de l'aldéhyde (IIa-4) dans 60 ml de dichloro-1,2-éthane on ajoute 2.42 .g (8.38 mmoles) de (4-aminométhyl)-4-fluoro-pipéridin-1-yl)-(3-chloro-4-fluorophényl)-méthanone (IIIa-1). Une fois le mélange homogène, on ajoute 5 g de tamis moléculaire 4 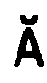 et le mélange est agité pendant 30 min. On additionne ensuite par fractions, 2.1 g de triacétoxy borohydrure de sodium (9.86 mmoles) et l'agitation est poursuivie pendant 2 heures à température ambiante. On additionne 10 ml de méthanol puis on filtre le mélange réactionnel. Le filtrat est concentré sous vide, l'huile obtenue est reprise dans du dichlorométhane, lavée à l'eau puis à l'eau salée, séchée sur Na₂SO₄, filtrée et concentrée sous pression réduite. Le résidu est purifié par chromatographie sur gel de silice (éluent : CH₂Cl₂ / CH₃OH 98 : 2). Le produit du titre est obtenu sous la forme d'une huile incolore (3 g).
¹HRMN (DMSOd₆) δ : 1.62-191 (m, 4 H); 2.33 (s, 1H); 2.67 (d, 2H); 3.05-3.39 (m, 3H); 3.75 (s, 5H); 4.25 (s, 1H); 5.07 (s, 2H); 6.94 (d, 2H); 7.32-7.51 (m, 6H); 7.66 (d, 1H); 8.24 (s, 1H).

### Etape 2: préparation du (3-Chloro-4-fluoro-phényl)-(4-fluoro-4-{[(5-hydroxy-pyridin-2-ylméthyl)-amino]-méthyl}-pipéridin-1-yl)-méthanone (1-1)

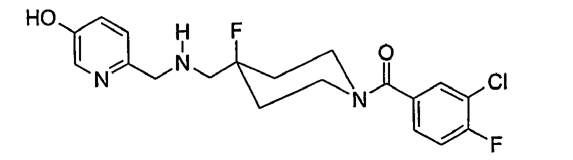

Dans une solution de 3 g (5.8 mmoles) du composé (1-1a) dans du dichlorométhane (50 ml) refroidi à 0°C et maintenue sous atmosphère inerte, on ajoute 4.5 ml d'acide trifluoroacétique (58 mmoles). Après retour à la température ambiante, le mélange est agité pendant 2 heures puis concentré sous vide. Le résidu est repris dans l'éther éthylique et le précipité formé filtré et lavé à l'éther éthylique. Le précipité est ensuite dissous dans de l'acétate d'éthyle et la solution extraite par une solution saturée de NaHCO₃. La phase organique est lavée à l'eau salée, séchée sur Na₂SO₄, filtrée et concentrée sous vide pour donner 2.2 g d'une huile jaune pâle.
¹HRMN (DMSOd₆) δ : 1.66-1.98 (m, 4H); 2.22 (s, 1H); 2.67 (d, 2H); 3.15-3.50 (m, 3H); 3.71 (s, 2H); 4.20 (s, 1H); 7.12 (dd, 1H); 7.20 (d, 1H); 7.42-7.51 (m, 2H); 7.66 (dd, 1H); 8.03 (d, 1H); 9.69 (s, 1H).

Fumarate du composé du titre :
F : 225-227 °C
C₂₃H₂₄ClF₂N₃O₆: 511.91
Calculé % : C 53.97; H 4.73; N 8.21
Trouvé % : C 53.73 ; H 4.97 ; N 8.01
¹HRMN (DMSOd₆) δ : 1.66 (m, 1H); 1.77 (m, 2H); 1.91 (m, 1H); 2.74 (d, 2H); 3.05 (m, 1H); 3.25 (m, 1H); 3.41 (m, 1H); 3.76 (s, 2H); 4.25 (m, 1H); 6.60 (s, 2H); 7.13 (dd, 1H); 7.23 (d, 1H); 7.45 (m, 2H); 7.66 (dd, 1H); 8.05 (d, 1H).

### Exemple comparatif 2 : préparation du (3-Chloro-4-fluoro-phényl)-(4-fluoro-4-{[(5-cyano-pyridin-2-ylméthyl)-amino]-méthyl}-pipéridin-1-yl)-méthanone (1-2)

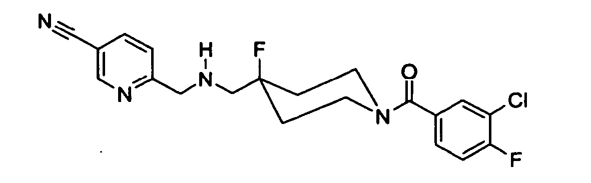

En procédant comme dans l'exemple 1 mais en remplaçant dans l'étape 1 l'aldéhyde (IIa-4) par le 6-formylnicotinonitrile (IIa-2) on obtient le composé du titre. Fumarate du composé du titre :
F : 170-172 °C
C₂₄H₂₃ClF₂N₄O₅ : 520.93
Calculé % : C 55.34; H 4.45; N 10.76
Trouvé % : C 55.32 ; H 4.50 ; N 10.73
¹HRMN (DMSOd₆) δ: 1.68 (m, 1H); 1.78 (m, 2H); 1.91 (m, 1H); 2.73 (d, 2H); 3.06 (m, 1 H); 3.26 (m, 1H); 3.40 (m, 1H); 3.94 (s, 2H); 4.26 (m, 1H); 6.62 (s, 2H); 7.47 (m, 2H); 7.67 (m, 2H); 8.27 (dd, 1 H); 8.94 (s, 1H).

### Exemple comparatif 3 : préparation du (3-Chloro-4-fluoro-phényl)-(4-fluoro-4-{[(5-chloro-pyridin-2-ylméthyl)-amino]-méthyl}-pipéridin-1-yl)-méthanone (1-3)

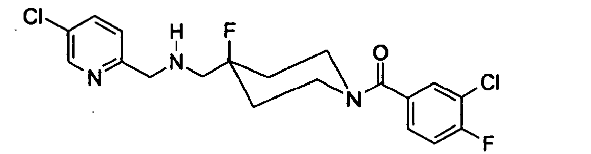

En procédant comme dans l'exemple 1 mais en remplaçant dans l'étape 1 l'aldéhyde (IIa-4) par le 5-chloro-pyridine-2-carbaldéhyde (IIa-5) on obtient le composé du titre.

Fumarate du composé du titre :
F : 160-162 °C
C₂₃H₂₃Cl₂F₂N₃O₄ : 530.35
Calculé % : C 52.09; H 4.37; N 7.92
Trouvé % : C 51.89 ; H 4.41 ; N 7.84
¹HRMN (DMSOd₆) δ : 1.65 (m, 1H); 1.78 (m, 2H); 1.78 (m, 1H); 2.72 (d, 2H); 3.06 (m, 1H); 3.25 (m, 1H); 3.39 (m, 1H); 3.85 (s, 2H); 4.25 (m, 1H); 6.62 (s, 2H); 7.44 (m, 1H); 7.49 (t, 2H); 7.66 (dd, 1H); 7.89 (dd, 1H); 8.54 (d, 1H).

### Exemple comparatif 4: préparation du (3-Chloro-4-fluoro-ph2nyl)-(4-fluoro-4-{[(5-fluorom2thyl-pyridin-2-ylm2thyl)-amino]-m2thyl} -pip2ridin-1-yl)-m2thanone (1-4)

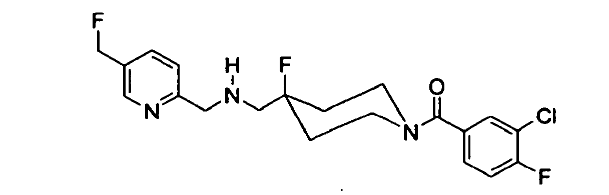

En procédant comme dans l'exemple 1 mais en remplaçant dans l'étape 1 l'aldéhyde (IIa-4) par le 5-fluorométhyl-pyridine-2-carbaldéhyde (IIa-7) on obtient le composé du titre.

Fumarate du composé du titre :
F : 157-159 °C
C₂₄H₂₅ClF₃N₃O₅ : 527.92
Calculé % : C 54.60; H 4.77; N 7.96
Trouvé % : C 54.42 ; H 4.65 ; N 7.75
¹HRMN (DMSOd₆) δ : 1.67 (m, 1H); 1.77 (m, 2H); 1.91 (m, 1H); 2.75 (d, 2H); 3.06 (m, 1H); 3.26 (m, 1H); 3.42 (m, 1H); 3.89 (s, 2H); 4.25 (m, 1H); 5.46 (d, 2H; J = 48 Hz); 6.61 (s, 2H); 7.44 (m, 1 H); 7.50 (d, 1H); 7.66 (dd, 1H); 7.85 (d, 1H); 8.57 (s, 1H).

### Exemple 5 : préparation du (3-Chloro-4-fluoro-phényl)-(4-fluoro-4-{[(5-méthyl-pyrimidin-2-ylméthyl)-amino]-méthyl}-pipéridin-1-yl)-méthanone (1-5)

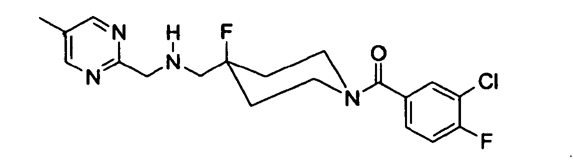

En procédant comme dans l'exemple 1 mais en remplaçant dans l'étape 1 l'aldéhyde (IIa-4) par le 5-méthyl-pyrimidine-2-carbaldehyde (IIb) on obtient le composé du titre.

Fumarate du composé du titre :
F : 105 °C (décomposition)
C₂₃H₂₅ClF₂N₄O₅ : 510.93
Calculé % : C 53.39; H 4.85; N 10.38
Trouvé % : C 53.20; H 5.11; N 10.52
¹HRMN (DMSOd₆) δ : 1.67-1.91 (m, 4H); 2.26 (s, 3H); 2.60 (d, 2H); 3.10-3.40 (m, 3H); 3.92 (s, 2H); 4.24 (s, 1H); 6.61 (s, 2H); 7.42-7.51 (m, 2H); 7.66 (d, 1H); 8.62 (s, 2H).

### Exemple 6: préparation du (3,4-Dichloro-phényl)-(4-fluoro-4-{[(5-méthyl-pyrimidin-2-ylméthyl)-amino]-méthyl}-pipéridin-1-yl)-méthanone (1-6)

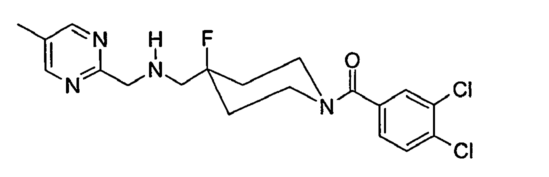

En procédant comme dans l'exemple 1 mais en remplaçant dans l'étape 1 l'aldéhyde (IIa-4) par le 5-méthyl-pyrimidine-2-carbaldéhyde (IIb) et l'amine primaire (IIIa-1) par la (4-aminométhyl-4-fluoro-pipéridin-1-yl)-(3,4-dichloro-phényl)-méthanone (IIIa-2) on obtient le composé du titre.

Hemi-fumarate du composé du titre :
F : 161 °C
C₂₁H₂₃Cl₂FN₄O₃ : 469.35
Calculé % : C 53.74; H 4.94; N 11.94
Trouvé % : C 53.54; H 4.95; N 12.06
¹HRMN (DMSOd₆) δ : 1.65-1.98 (m, 4H); 2.25 (s, 3H); 2.80 (d, 2H); 3.07-3.51 (m, 3H); 3.89 (s, 2H); 4.24 (s, 1H); 6.61 (s, 1H); 7.40 (d, 1H); 7.70 (s, 1H); 7.71 (d, 1H); 8.62 (s, 2H).

### Exemple 7 : préparation du (3-Chloro-4-fluoro-ph2nyl)-(4-fluoro-4-{[(6-m2thyl-pyridazin-3-ylméthyl)-amino]-méthyl}-pipéridin-1-yl)-méthanone (1-7)

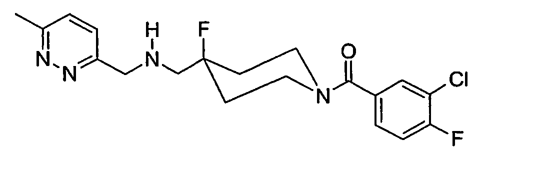

En procédant comme dans l'exemple 1 mais en remplaçant dans l'étape 1 l'aldéhyde (IIa-4) par le 6-méthyl-pyridazine-3-carbaldéhyde (IIc) on obtient le composé du titre.

Dichlorhydrate du composé du titre :
F : 205 °C (décomposition)
C₁₉H₂₃C1₃F₂N₄O : 467.78
Calculé % : C 48.79; H 4.96; N11.98
Trouvé % : C 48.60; H 4.92; N 11.88
¹HRMN (DMSOd₆) δ : 1.79-2.04 (m, 4H); 2.68 (s, 3H); 3.07-3.51 (m, 3H); 3.35 (d, 2H); 4.26 (s, 1H); 4.53 (s, 2H); 7.43-7.46 (m, 1H); 7.52 (t, 1H); 7.67 (d, 1H); 7.76 (d, 1H); 7.92 (d, 1H).

### Exemple 8: préparation du (3,4-dichloro-phényl)-(4-fluoro-4-{[(6-méthyl-pyridazin-3-ylméthyl)-amino]-méthyl}-pipéridin-1-yl)-méthanone (1-8)

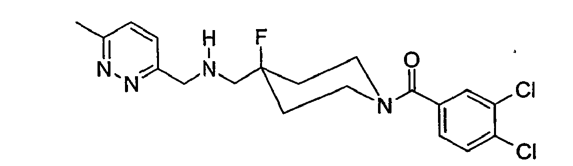

En procédant comme dans l'exemple 1 mais en remplaçant dans l'étape 1 l'aldéhyde (IIa-4) par le 6-méthyl-pyridazine-3-carbaldéhyde (IIc) et l'amine primaire (IIIa-1) par la (4-aminométhyl-4-fluoro-pipéridin-1-yl)-(3,4-dichloro-phényl)-méthanone (IIIa-2) on obtient le composé du titre.

Oxalate du composé du titre :
F : 203 °C (décomposition)
C₂₁H₂₃Cl₂FN₄O₅ : 501.35
Calculé % : C 50.31; H 4.62; N 11.18
Trouvé % : C 50.34; H 4.69; N 11.14
¹HRMN (DMSOd₆) δ : 1.68-1.96 (m, 4H); 2.61 (s, 3H); 2.94 (d, 2H); 3.05-3.57 (m, 3H); 4.19 (s, 2H); 4.28 (s, 1H); 7.39 (d, 1H); 7.58 (d, 1H); 7.65 (d, 1H); 7.69-7.73 (m, 2H).

### Exemple comparatif 9: préparation du (3-Chloro-4-fluoro-phényl)-(4-chloro-4-{[(5-méthyl-pyridin-2-ylméthyl)-amino]-méthyl}-pipéridin-1-yl)-méthanone (1-9)

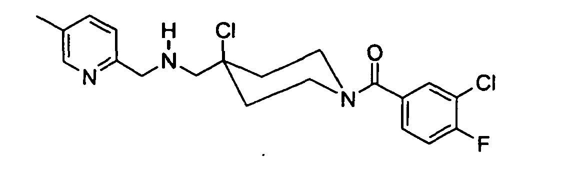

En procédant comme dans l'exemple 1 mais en remplaçant dans l'étape 1 l'aldéhyde (IIa-4) par le 5-méthyl-pyridine-2-carbaldéhyde (IIa-1) et l'amine primaire (IIIa-1) par la (4-aminométhyl-4-chloro-pipéridin-1-yl)-(3-chloro-4-fluoro-phényl)-méthanone (IIIb-1) on obtient le composé du titre.

Dichlorhydrate du composé du titre :
F : 190 °C (décomposition)
C₂₀H₂₄Cl₄FN₃O : 483.35
Calculé % : C 49.71; H 5.01; N 8.70
Trouvé % : C 49.62; H 4.99; N 8.72
¹HRMN (DMSOd₆) δ : 1.85-2.01 (m, 4H); 2.34 (s, 3H); 3.07-3.55 (m, 3H); 4.18 (s, 1H); 4.37 (s, 2H); 7.40-7.54 (m, 3H); 7.67 (d, 1H); 7.74 (d, 1H).

### Exemple comparatif 10 : préparation du (4-Fluoro-phényl)-(4-fluoro-4-{[(5-méthyl-pyridin-2-ylméthyl)-amino]-méthyl}-pipéridin-1-yl)-méthanone (1-10)

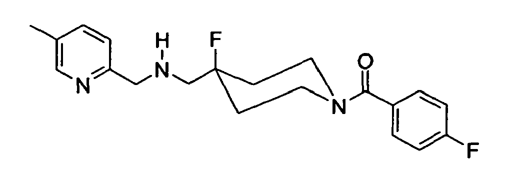

En procédant comme dans l'exemple 1 mais en remplaçant dans l'étape 1 l'aldéhyde (IIa-4) par le 5-méthyl-pyridine-2-carbaldéhyde (IIa-1) et l'amine primaire (IIIa-1) par la (4-aminométhyl-4-fluoro-pipéridin-1-yl)-(4-fluoro-phényl)-méthanone (IIIa-3) on obtient le composé du titre.

Fumarate du composé du titre :
F:154-156°C
C₂₄H₂₇F₂N₃O₅ : 475.49
¹HRMN (DMSOd₆) δ : 1.66 (m, 1H); 1.77 (m, 2H); 1.86 (m, 1H); 2.27 (s, 3H); 2.75 (d, 2H); 3.08 (m, 1H); 3.23 (m, 1H); 3.43 (m, 1H); 3.83 (s, 2H); 4.26 (m, 1H); 6.42 (s, 2H); 7.29 (m, 3H); 7.48 (dd, 2H); 7.57 (d, 1H); 8.34 (s, 1H).

### Exemple comparatif 11 : préparation du (3,4-Difluoro-phényl)-(4-fluoro-4-{[(5-méthyl-pyridin-2-ylméthyl)-amino]-méthyl}-pipéridin-1-yl)-méthanone (1-11)

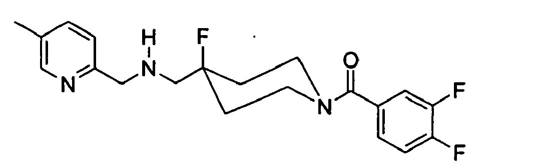

En procédant comme dans l'exemple 1 mais en remplaçant dans l'étape 1 l'aldéhyde (IIa-4) par le 5-méthyl-pyridine-2-carbaldéhyde (IIa-1) et l'amine primaire (IIIa-1) par la (4-aminométhyl-4-fluoro-pipéridin-1-yl)-(3,4-difluoro-phényl)-méthanone (IIIa-4) on obtient le composé du titre.

Fumarate du composé du titre :
F:158°C
C₂₄H₂₆F₃N₃O₅ : 493.49
Calculé % : C 58.41; H 5.31; N 8.52
Trouvé % : C 58.45; H 5.35; N 8.41
¹HRMN (DMSOd₆) δ : 1.64-1.91 (m, 4H); 2.27 (s, 3H); 2.73 (d, 2H); 3.06-3.41 (m, 3H); 3.82 (s, 2H); 6.61 (s, 2H); 7.26-7.31 (m, 1H); 7.31 (d, 1H); 7.48-7.58 (m, 3H); 8.33 (s, 1H).

### Exemple comparatif 12 : préparation du (3-Fluoro-4-chloro-phényl)-(4-fluoro-4-{[(5-méthyl-pyridin-2-ylméthyl)-amino]-méthyl}-pipéridin-1-yl)-méthanone (1-12)

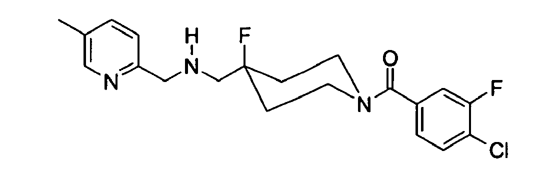

En procédant comme dans l'exemple 1 mais en remplaçant dans l'étape 1 l'aldéhyde (IIa-4) par le 5-méthyl-pyridine-2-carbaldéhyde (IIa-1) et l'amine primaire (IIIa-1) par la (4-aminométhyl-4-fluoro-pipéridin-1 -yl)-(3-fluoro-4-chloro-phényl)-méthanone (IIIa-5) on obtient le composé du titre.

Fumarate du composé du titre :
F : 150 °C
C₂₄H₂₆ClF₂N₃O₅ : 509.94
Calculé % : C 56.53; H 5.14; N 8.24
Trouvé % : C 56.58; H 5.24; N 8.19
¹HRMN (DMSOd₆) 8 : 1.64-1.92 (m, 4H); 2.27 (s, 3H); 2.72 (d, 2H); 3.05-3.51 (m, 3H); 3.82 (s, 2H); 4.25 (s, 1H); 6.61 (s, 2H); 7.26-7.32 (m, 2H); 7.53 (d, 1H); 7.56 (d, 1H); 7.67 (m, 1H); 8.33 (s, 1H).

### Exemple comparatif 13 : préparation du (3-Cyano-4-fluoro-phényl)-(4-fluoro-4-{[(5-méthyl-pyridin-2-ylméthyl)-amino]-méthyl}-pipéridin-1-yl)-méthanone (1-13)

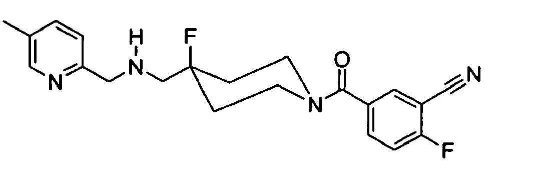

En procédant comme dans l'exemple 1 mais en remplaçant dans l'étape 1 l'aldéhyde (IIa-4) par le 5-méthyl-pyridine-2-carbaldéhyde (IIa-1) et l'amine primaire (IIIa-1) par la (4-aminométhyl-4-fluoro-pipéridin-1-yl)-(3-cyano-4-fluoro-phényl)-méthanone (IIIa-6) on obtient le composé du titre.

Fumarate du composé du titre :
F:175°C
C₂₅H₂₆F₂N₄O₅ : 500.51
Calculé % : C 59.99; H 5.24; N11.19
Trouvé % : C 59.99; H 5.32; N 10.85
¹HRMN (DMSOd₆) δ : 1.69-1.81 (m, 4H); 2.29 (s, 3H); 2.83 (d, 2H); 3.28-3.48 (m, 3H); 3.91 (s, 2H); 4.28 (s, 1H); 6.61 (s, 2H); 7.34 (d, 1H); 7.58-7.63 (m, 2H); 7.82-7.87 (m, 1H); 8.04 (m, 1H); 8.36 (s, 1H).

### Exemple comparatif 14 : préparation du (3-Trifluorométhyl-phényl)-(4-fluoro-4-{[(5-méthyl-pyridin-2-ylméthyl)-amino]-méthyl}-pipéridin-1-yl)-méthanone (1-14)

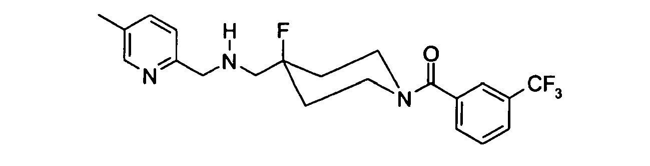

En procédant comme dans l'exemple 1 mais en remplaçant dans l'étape 1 l'aldéhyde (IIa-4) par le 5-méthyl-pyridine-2-carbaldéhyde (IIa-1) et l'amine primaire (IIIa-1) par la (4-aminométhyl-4-fluoro-pipéridin-1-yl)-(3-trifluorométhyl-phényl)-méthanone (IIIa-7) on obtient le composé du titre.

Dichlorhydrate du composé du titre :
F : 172 °C (sublimation)
C₂₁H₂₅Cl₂F₄N₃O : 482.35
Calculé % : C 52.29; H 5.22; N 8.71
Trouvé % : C 52.56; H 5.47; N 8.51
¹HRMN (DMSOd₆) δ : 1.83-2.08 (m, 4H); 2.37 (s, 3H); 3.08-3.51 (m, 3H); 3.28 (d, 2H); 4.36 (s, 1H); 4.37 (s, 2H); 7.66-7.88 (m, 6H); 8.57 (s, 1H).

### ETUDE PHARMACOLOGIQUE DES PRODUITS DE L'INVENTION

### Mesure de l'affinité des composés de l'invention pour les récepteurs 5-HT_{1A}

### PROTOCOLE

L'affinité in vitro des composés de l'invention pour les récepteurs 5-HT_{1A} a été déterminée par la mesure du déplacement de la [³H]8-OH-DPAT (TRK 850 ; 160-240 Ci/mmole).

L'étude de la liaison au récepteur 5-HT_{1A} est réalisée comme décrit par Sleight et Peroutka (Naunyn-Schmiedeberg's Arch. Pharmaco. 1991, 343, 106). Pour ces expérimentations, des cortex cérébraux de rat sont utilisés. Après décongélation du cerveau dans du tampon Tris-HCl (50 mmoles, pH = 7,4) à 25°C, le cortex cérébral est prélevé et homogénéisé dans 20 volumes de tampon maintenu à 4°C. L'homogénat est centrifugé à 39 000 g pendant 10 min, le culot de centrifugation est mis en suspension dans le même volume de tampon et centrifugé à nouveau. Après une nouvelle mise en suspension dans les mêmes conditions, l'homogénat est incubé pendant 10 min à 37°C puis centrifugé à nouveau. Le culot final est mis en suspension dans du tampon de réaction froid Tris-HCl (50 mmoles) à 25°C contenant 10 mmoles de pargyline, 4 mmoles de CaCl₂ et 0,10 % d'acide ascorbique. La concentration finale de tissu dans le milieu d'incubation est de 10 mg/tube.

Les tubes de réaction contiennent 0.10 ml de [³H]8-OH-DPAT (0.20 mmoles en final), 0.10 ml de produit à tester 6-7 concentrations et 0.80 ml de tissu. La liaison non spécifique est définie en utilisant 10 mmoles de sérotonine. Les tubes de réaction sont incubés à 23°C pendant 30 min puis leur contenu est rapidement filtré sous vide sur filtres Whatman GFB, les tubes sont rincés avec 2 fois 5 ml de tampon Tris-HCl à 25°C. La radioactivité recueillie sur le filtre est analysée en scintillation liquide en ajoutant 4 ml de liquide scintillant (Emulsifier Safe, Packard, Warrenville, USA). Toutes les expériences sont réalisées en triple.

### Mesure de l'affinité des composés de l'invention pour les récepteurs D₂.

### PROTOCOLE

L'affinité in vitro des composés de l'invention pour les récepteurs dopaminergiques D₂, a été déterminée par la mesure du déplacement du [³H]YM-09151-2 (NET-1004 70-87 Ci/mmole). L'étude de la liaison au récepteur D₂ est réalisée comme décrit par Niznik (Naunyn-Schmiedeberg's Arch. Pharmacol. Methods 1985, 329, 333). Pour ces expérimentations, on utilise le striatum de rat. Après décongélation du cerveau dans du tampon Tric-HCl (50 mmoles, pH = 7.4) à 25°C, le striatum est prélevé et homogénéisé dans 40 volumes de tampon maintenu à 4°C. L'homogénat est centrifugé à 20 000 g pendant 10 min, le culot de centrifugation est mis en suspension dans le même volume de tampon et centrifugé à nouveau. Le culot final est mis en suspension dans du tampon de réaction froid Tris-HCl à 25°C contenant 120 mmoles de NaCl et 5 mmoles de KCl. La concentration finale de tissu dans le milieu d'incubation est de 2 mg/tube. Les tubes de réaction contiennent 0,20 ml de [³H]YM-09151-2 (0.05 mmoles en final), 0,20 ml de produit à tester 6-7 concentrations et 1,60 ml de tissu. La liaison non spécifique est définie en utilisant 1 mmole de (+)-Butaclamol. Les tubes de réaction sont incubés à 23°C pendant 60 min puis leur contenu est rapidement filtré sur filtres Whatman GF/B, les tubes sont rincés 2 fois avec 5 ml de tampon Tris-HCl à 25°C. La radioactivité recueillie sur le filtre est analysée en scintillation liquide en ajoutant 4 ml de liquide scintillant (Emulsifier Safe, Packard). Toutes les expériences sont réalisées en triple.

Les constantes d'inhibition (Ki) des produits de l'invention sont estimées à partir des expérimentations de déplacement en utilisant le programme de régression non-linéaire RADLIG version 4 de EBDA (Equilibrium Binding Data Analysis) (Biosoft, Cambridge, UK, Mc Pherson, 1985). Les constantes de dissociation des ligands radioactifs utilisées dans les calculs sont de 0,31 mmoles pour [³H]8-OH-DPAT et de 0.036 mmoles pour [³H]YM-09151-2. Les valeurs de pKi (-logKi) sont données sous forme de moyenne d'au moins 3 expérimentations.

### Mesure de l'efficacité des composés de l'invention

### PROTOCOLE

Les cellules (Gibco Biocult. Laboratory, Paisley UK) sont prélevées dans du tampon phosphate (pH = 7,4) et centrifugées à 48 000g pendant 20 min. Le culot de centrifugation est homogénéisé dans de l'Hepes (20 mmoles, pH = 7.4) contenant de l'EDTA (10 mmoles) et centrifugé à nouveau à 48 000g pendant 10 min. Le culot de centrifugation est lavé 2 fois dans l'Hepes (10 mmoles, pH = 7.4) contenant de l'EDTA (0,1 mmoles). Le culot est conservé à -80°C par fractions de 600 à 750 µg de protéine. Le culot est dilué 20 fois dans de l'Hepes (20 mmoles) contenant 30 µmoles de GDP, 100 mmoles de NaCl, 3 mmoles de MgCl₂ et 0,2 mmoles d'acide ascorbique. Le milieu d'incubation contient 0,4 ml de préparation membranaire et 0,05 ml du composé à tester. Après 30 min d'incubation à 25°C, 0.05 ml de [³⁵S]GTPγS (500 picomoles), ([³⁵S]GTPγS (1100 Ci/mmole), Amersham, Les Ulis France), est ajouté et le mélange incubé pendant 30 min. Les réactions sont arrêtées par addition de 3 ml d'Hepes glacé (20 mmoles) contenant 3 mmoles de MgCl₂ et filtration rapide sur filtres Whatman GF/B. Les tubes sont rincés 3 fois avec 5 ml de tampon Hepes à 25°C. La radioactivité recueillie sur le filtre est analysée en scintillation liquide en ajoutant 4 ml de liquide scintillant (Emulsifier Safe, Packard). La liaison non spécifique est déterminée en présence de GTPγS froid. La stimulation maximale de la liaison du [³⁵S]GTPγS est définie par la sérotonine (10 µmoles). Toutes les expériences sont réalisées en triple.

Les composés de l'invention ont été comparés à la sérotonine, la buspirone, à la 8-OH-DPAT et au composé I-66 (WO 98/22459).

### RESULTATS

Les composés de formule (1) ainsi que leurs sels thérapeutiquement acceptable présentent des propriétés pharmacologiques intéressantes. Les résultats des essais sont regroupés dans le tableau suivant :

| Composé | 5-HT1_{A} pKi | D₂ pKi | Sélectivité 5-HT1_{A}/D₂ | % stimulation Effet Maximum |
|---|---|---|---|---|
| 5-HT | - | - | - | 100 |
| 8-OH-DPAT | 8.8 | 6.2 | ~ 350 | 41 |
| (+)-8-OH-DPAT | 8.7 | 6.1 | ~ 350 | 59 |
| buspirone | 7.6 | 7.4 | ~ 1 | 22 |
| I-66 | 9.1 | <5 | >1000 | 75 |
| 1-7 | 8.3 | <5 | >1000 | 90 |
| 1-9 | 9.1 | <5 | >1000 | 100 |

Les résultats des essais montrent que les composés de formule (1) possèdent une affinité élevée pour les récepteurs sérotoninergiques du sous-type 5-HT_{1A} et qu'ils sont sélectifs pour ces récepteurs vis à vis des récepteurs D₂.

La capacité des composés de formule (1) à stimuler la liaison de [³⁵S]GTPγS dans une préparation de membrane cellulaire est, de plus, très supérieure à celles des composés de référence tels que la 8-OH-DPAT, la (+)-8-OH-DPAT et la buspirone. L'efficacité de certains composés de l'invention est aussi significativement supérieure à celle du composé (I-66) qui est l'agoniste le plus efficace décrit dans l'art antérieur. Nous constatons également que l'efficacité de certains composés de l'invention est proche voire indiscernable de celle de la sérotonine (5-HT).

Il ressort donc de cette étude, que les composés de l'invention ont l'avantage de présenter un profil d'agonistes 5-HT1_{A} sélectifs et plus efficaces que les produits antérieurs. A ce titre, les composés de l'invention sont potentiellement utiles dans le traitement des désordres, troubles ou pathologies impliquant des dysfonctionnements sérotoninergiques tels que, par exemple, la dépression, la perception de la douleur et la dépendance à certaines substances.

L'administration des composés de l'invention peut être réalisée par voie orale, nasale, sublinguale, rectale ou parentérale. A titre d'exemples de formulation non limitatifs, nous donnons ci-après, une préparation des composés de l'invention. Les ingrédients ainsi que d'autres, thérapeutiquement acceptables, peuvent être introduits en d'autres proportions sans modifier la portée de l'invention. Les termes 'ingrédient actif utilisés dans l'exemple de formulation ci-après font références à un composé de formule (1) ou un sel d'addition ou éventuellement un hydrate d'un sel d'addition du composé de formule (1) avec un acide minéral ou un acide organique pharmaceutiquement acceptable.

### Exemple de composition pharmaceutique

Formule de préparation pour 1000 comprimés contenant chacun 10 mg de l'ingrédient actif :

| | |
|---|---|
| Ingrédient actif | 10 g |
| Lactose | 100 g |
| Amidon de blé | 10 g |
| Stéarate de magnésium | 3 g |
| Talc | 3 g |

## Revendications

1. Composés de formule générale (1) : 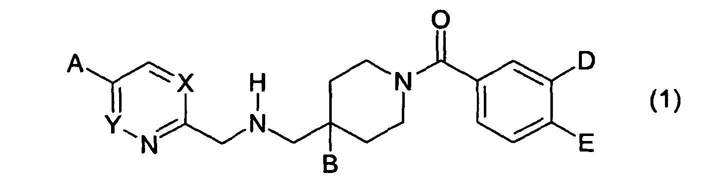 dans laquelle :
X représente un atome de carbone lié à un atome d'hydrogène (CH) et Y représente un atome d'azote ;
ou Y représente un atome de carbone lié à un atome d'hydrogène (CH) et X représente un atome d'azote ;
A représente un radical méthyle (CH₃), fluorométhyle (CH₂F), cyano (CN), hydroxyle (OH), méthoxyle (OCH₃), un atome de chlore ou un atome de fluor ;
B représente un atome de chlore ou un atome de fluor ;
D représente un atome d'hydrogène, un atome de chlore, un atome de fluor, un groupe cyano (C≡N) ou un groupe trifluorométhyle (CF₃) ;
E représente un atome d'hydrogène, de fluor ou de chlore ;
leurs sels d'addition et éventuellement les hydrates des sels d'addition avec les acides minéraux ou les acides organiques pharmaceutiquement acceptables ainsi que leurs formes tautomères.

2. Composés de formule générale (1) selon la revendication 1, dans laquelle :
B et E représentent chacun un atome de fluor, et
D représente un atome de chlore.

3. Dérivés selon la revendication 1, **caractérisé en ce qu'**ils sont choisis parmi les composés suivants :
(3-Chloro-4-fluoro-phényl)-(4-fluoro-4- {[(5-méthyl-pyrimidin-2-ylméthyl)-amino]-méthyl}-pipéridin-1-yl)-méthanone ;
(3,4-Dichloro-phényl)-(4-fluoro-4-{[(5-méthyl-pyrimidin-2-ylméthyl)-amino]-méthyl}-pipéridin-1-yl)-méthanone ;
(3-Chloro-4-fluoro-phényl)-(4-fluoro-4-{[(6-méthyl-pyridazin-3-ylméthyl)-amino]-méthyl}-pipéridin-1-yl)-méthanone ;
(3,4-dichloro-phényl)-(4-fluoro-4-{[(6-méthyl-pyridazin-3-ylméthyl)-amino]-méthyl}-pipéridin-1-yl)-méthanone ;
leurs sels d'addition et éventuellement les hydrates des sels d'addition avec les acides minéraux ou les acides organiques pharmaceutiquement acceptables ainsi que leurs formes tautomères.

4. Composés selon l'une des revendication 1 à 3 à titre de médicaments.

5. Compositions pharmaceutiques **caractérisées en ce qu'**elles contiennent comme ingrédient actif au moins un composé selon l'une des revendications 1 à 3 associé à un support pharmaceutique inerte ou autres véhicules pharmaceutiquement acceptables et éventuellement à un autre médicament.

6. Utilisation d'un composé selon l'une des revendications 1 à 3 pour la fabrication d'un médicament utile pour le traitement de la maladie de la dépression.

7. Utilisation d'un composé selon l'une des revendications 1 à 3 pour la fabrication d'un médicament utile pour le traitement de la douleur.

8. Utilisation d'un composé selon l'une des revendications 1 à 3 pour la fabrication d'un médicament utile pour le traitement de la dépendance à certaines substances.

## Claims

1. Compounds of general formula (1); 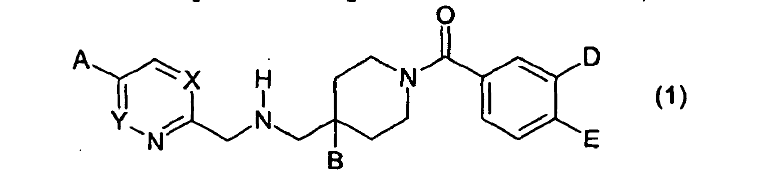 wherein:
X represents a carbon atom bound to a hydrogen atom (CH) and Y represents a nitrogen atom;
or Y represents a carbon atom bound to a hydrogen atom (CH) and X represents a nitrogen atom;
A represents a methyl (CH₃), fluoromethyl (CH₂F), cyano (CN), hydroxyl (OH), methoxyl (OCH₃) radical, a chlorine atom or a fluorine atom;
B represents a chlorine atom or a fluorine atom;
D represents a hydrogen atom, a chlorine atom, a fluorine atom, a cyano (C≡N) group or a trifluoromethyl group (CF₃);
E represents a hydrogen, fluorine or chlorine atom; their addition salts and optionally hydrates of addition salts with pharmaceutically acceptable mineral acids or organic acids as well as their tautomeric forms.

2. The compounds of general formula (1) according to claim 1, wherein:
B and E each represent a fluorine atom, and
D represents a chlorine atom.

3. The derivatives according to claim 1, **characterized in that** they are selected from the following compounds:
(3-chloro-4-fluoro-phenyl)-(4-fluoro-4-{[(5-methyl-pyrimidin-2-ylmethyl)-amino]-methyl}-piperidin-1-yl)-methanone;
(3,4-dichloro-phenyl)-(4-fluoro-4-{[(5-methyl-pyrimidin-2-ylmethyl)-amino]-methyl}-piperidin-1-yl)-methanone;
(3-chloro-4-fluoro-phenyl)-(4-fluoro-4-{[(6-methyl-pyridazin-3-ylmethyl)-amino]-methyl}-piperidin-1-yl)-methanone;
(3,4-dichloro-phenyl)-4-fluoro-4-{[(6-methyl-pyridazin-3-ylmethyl)-amino]-methyl}-piperidin-1-yl)-methanone; their addition salts and optionally hydrates of addition salts with pharmaceutically acceptable mineral acids of organic acids as well as their tautomeric forms.

4. The compounds according to any of claims 1 to 3 as drugs.

5. Pharmaceutical compositions **characterized in that** they contain as active ingredient at least one compound according to any of claims 1 to 3, associated with an inner pharmaceutical support or other pharmaceutically acceptable carriers and optionally with another drug.

6. The use of a compound according to any of claims 1 to 3, for making a drug useful for treating the depression disease.

7. The use of a compound according to any of claims 1 to 3, for making a drug useful for treating pain.

8. The use of a compound according to any of claims 1 to 3, for making a drug useful for treating addiction to certain substances.

## Patentansprüche

1. Verbindungen der allgemeinen Formel (1) 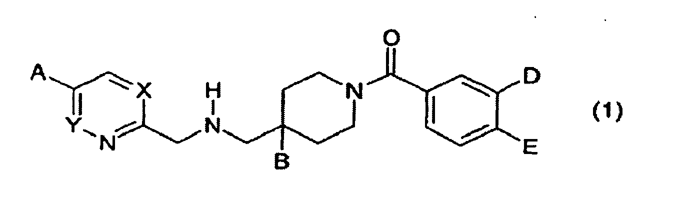 wobei:
X ein mit einem Wasserstoffatom verbundenes Kohlenstoffatom (CH) darstellt und Y ein Stickstoffatom darstellt,
oder Y ein mit einem Wasserstoffatom verbundenes Kohlenstoffatom (CH) darstellt und X ein Stickstoffatom darstellt,
A ein Ethyl- (CH₃), Fluormethyl- (CH₂F), Cyano-(CN),- Hydroxyl- (OH), Methoxylradikal (OCH₃), ein Chloratom oder ein Fluoratom darstellt,
B ein Chloratom oder ein Fluoratom darstellt,
D ein Wasserstoffatom, ein Chloratom, ein Fluoratom, eine Cyanogruppe (C≡N) oder eine Trifluormethylgruppe (CF₃) darstellt,
E ein Wasserstoff-, Fluor- oder Chloratom darstellt,
ihre Additionssalze und eventuell die Hydrate der Additionssalze mit den Mineralsäuren oder den pharmazeutisch akzeptablen organischen Säuren sowie ihren Tautomerformen.

2. Verbindungen der allgemeinen Formel (1) nach Anspruch 1, wobei:
B und E jeweils ein Fluoratom darstellen, und
D ein Chloratom darstellt.

3. Derivate nach Anspruch 1, **dadurch gekennzeichnet, dass** sie aus den folgenden Verbindungen ausgewählt sind:
(3-Chloro-4-fluoro-phenyl)-(4-fluoro-4-{[(5-methyl-pyrimidin-2-ylmethyl)-amino]-methyl}-piperidin-1-yl)-methanon,
(3,4-Dichloro-phenyl)-(4-fluoro-4-{[(5-methyl-pyrimidin-2-ylmethyl)-amino]-methyl}-piperidin-1-yl)-methanon,
(3-Chloro-4-fluoro-phenyl)-(4-fluoro-4-{[(6-methyl-pyridazin-3-ylmethyl)-amino]-methyl}-piperidin-1-yl)-methanon,
(3,4-Dichloro-phenyl)-(4-fluoro-4-{[(6-methyl-pyridazin-3-ylmethyl)-amino]-methyl}-piperidin-1-yl)-methanon,
ihren Additionssalzen und eventuell den Hydraten der Additionssalze mit den Mineralsäuren oder den pharmazeutisch akzeptablen organischen Säuren sowie ihren Tautomerformen.

4. Verbindungen nach einem der Ansprüche 1 bis 3 als Arzneimittel.

5. Pharmazeutische Zusammensetzungen, **dadurch gekennzeichnet, dass** sie als Wirkstoff mindestens eine Verbindung nach einem der Ansprüche 1 bis 3 in Kombination mit einem inerten pharmazeutischen Träger oder einer anderen pharmazeutisch akzeptablen Trägersubstanz und eventuell mit einem anderen Arzneimittel enthalten.

6. Verwendung einer Verbindung nach einem der Ansprüche 1 bis 3 zur Herstellung eines Arzneimittels zur Behandlung der Depressionskrankheit.

7. Verwendung einer Verbindung nach einem der Ansprüche 1 bis 3 zur Herstellung eines Arzneimittels zur Schmerzbehandlung.

8. Verwendung einer Verbindung nach einem der Ansprüche 1 bis 3 zur Herstellung eines Arzneimittels zur Behandlung der Abhängigkeit von bestimmten Substanzen.
